# EUROPEAN PATENT APPLICATION

(11) **EP 1 748 294 A2**
(43) Date of publication of application: **31.01.2007**
(21) Application number: 06115620.4
(22) Date of filing: 19.06.2006
(51) Int. Cl.: G01N 33/48, A01K 67/027

(54) **In vivo model for immunocomparability**

(30) Priority: 30.06.2005 EP 05105946
(71) Applicant: F. HOFFMANN-LA ROCHE AG, 4070 Basel (CH)
(72) Inventor: Beck, Hermann, 79539 Loerrach (DE); Iglesias, Antonio, 79102 Freiburg i.Br. (DE); Schreitmueller, Thomas, 4147 Aesch (CH); Zocher, Marcel, 79539 Loerrach (DE)

(57) **Abstract**

The present invention relates to methods for determining the relative immunogenicity of exogenous antibody variants comprising a) creating a non-human animal, which is transgenic for a standard antibody, b) contacting said transgenic non-human animal with an exogenous antibody whereby said exogenous antibody is a variant of said transgenic standard antibody of a), and c) determining the immune response of the transgenic non-human animal elicited by the exogenous antibody of (b).

## Description

The present invention relates to an *in vivo* model useful for assessment of the relative immunogenicity of different preparations of an antibody-based therapeutic or diagnostic. Immunogenicity is compared by a method comprising the steps of a) creating a non-human animal, which is transgenic for an antibody, b) contacting said transgenic non-human animal with an exogenous antibody whereby said exogenous antibody is a standard preparation of the transgenic antibody of (a) or a variant thereof, and c) determining the immune response of the transgenic non-human animal elicited by the exogenous antibody of (b).

Therapeutic monoclonal antibodies were introduced into clinical practice in 1986 with the approval of the recombinant murine antibody Orthoclone, OKT-3, indicated for the prevention of allograft rejection in renal transplantation *[*Proceedings of an international symposium on monoclonal antibody therapy with orthoclone OKT3 in renal transplantation. Transplant Proc, 1986. 18(4): p. 923-56.Burdick, J.F., et al., Reversal of progressive renal allograft dysfunction with OKT3. Nephron, 1987. 46 Suppl 1: p. 52-5; Farges, O., et al., A randomized trial of OKT3-based versus cyclosporine-based immunoprophylaxis after liver transplantation. Long-term results of a European and Australian multicenter study. Transplantation, 1994. 58(8): p. 891-8.; Farrell, M.L., Orthoclone OKT3: a treatment for acute renal allograft rejection. Anna J, 1987. 15(6): p. 373-6.: Friedman, J., et al., Orthoclone OKT3 treatment of acute renal allograft rejection. Transplant Proc, 1987. 19(2 Suppl 2): p. 46; Gordon, R.D., et al., Experience with Orthoclone OKT3 monoclonal antibody in liver transplantation. Am J Kidney Dis, 1988. 11(2): p. 141-4; Smith, S.L., Ten years of Orthoclone OKT3 (muromonab-CD3): a review. J Transpl Coord, 1996. 6(3): p. 109-19; quiz 120-1]. ReoPro was approved in 1994 by the FDA [Tcheng, J.E., Dosing and administration of ReoPro (c7E3 Fab). J Invasive Cardiol, 1994. 6 Suppl A: p. 29A-33A; discussion 51A-54A; Faulds, D. and E.M. Sorkin, Abciximab (c7E3 Fab). A review of its pharmacology and therapeutic potential in ischaemic heart disease. Drugs, 1994. 48(4): p. 583-98; Huston, J.S. and A.J. George, Engineered antibodies take center stage. Hum Antibodies, 2001. 10(3-4): p. 127-42], an indication that acceptance of the therapeutic as well as commercial potential of therapeutic antibodies by the pharmaceutical industry was initially slow. Technical challenges associated with the large-scale manufacture of biotherapeutic agents, early disappointments in the treatment of cancer, as well as concerns about immune reactions against the therapeutic agent arising in humans largely explain the delayed expansion of these novel therapeutic agents.

Many of the early challenges associated with therapeutic antibodies have recently been overcome by improvements in antibody generation, development and manufacturing technologies [Kipriyanov, S.M. and F. Le Gall, Generation and production of engineered antibodies. Mol Biotechnol, 2004. 26(1): p. 39-60]. In 2002, there were 13 monoclonal antibody-based therapeutics approved in the United States, with another 400 products in clinical testing [Gura, T., Therapeutic antibodies: magic bullets hit the target. Nature, 2002. 417(6889): p. 584-6]. However, there is still limited information available regarding the immunogenic response generated against many of these agents [Schellekens, H., Bioequivalence and the immunogenicity of biophartnaceuticals. Nat Rev Drug Discov, 2002. 1(6): p. 457-62; Schellekens, H., When biotech proteins go off-patent. Trends Biotechnol, 2004. 22(8): p. 406-10].

In contrast to small-molecule drugs, therapeutic antibodies are large molecules with a highly complex three-dimensional structure. While synthetic drugs can be well-characterized using sensitive physicochemical analytical methods, recombinant proteins cannot be fully characterized using physicochemical methods alone. Recombinant biological proteins are secreted by genetically modified cells, which are inherently variable. The product may be produced as different isomers, or it may form aggregates. Glycosylation and other post-translational modifications are other complications that lead to heterogeneity. Manufacturing processes for biotherapeutics are far more complex than the process used for synthetic drug products. Thus, the final properties of the biological therapeutic protein are highly dependent on the production process [Rosenberg, A.S., Immunogenicity of biological therapeutics: a hierarchy of concerns. Dev Biol (Basel), 2003. 112: p. 15-21] and may also be influenced by the formulation. Batch-to-batch variations are common, and this makes reproducibility one of the criteria by which the quality of the product is assessed. Since physicochemical methods are unable to fully characterize a biotherapeutic product, biological systems are needed to characterize the product in complementation to physicochemical data [Schellekens, H., Bioequivalence and the immunogenicity of biopharmaceuticals. Nat Rev Drug Discov, 2002. 1(6): p. 457-62]. Ensuring that different production batches are comparable in their inherent immunological properties has been one of the most difficult challenges faced in the manufacturing of biopharmaceuticals.

Most therapeutic proteins induce an immune response when administered to patients [Ryff, J.C. and H. Schellekens, Immunogenicity of rDNA-derived pharmaceuticals. Trends Pharmacol Sci, 2002. 23(6): p. 254-6], and therapeutic antibodies are no exception [Ryff, J.C. and H. Schellekens, Immunogenicity of rDNA-derzved pharmaceuticals. Trends Pharmacol Sci, 2002. 23(6): p. 254-6]. However, anti-product antibody incidence differs widely among products, with some inducing antibodies in most individuals, while for others the incidence of an immune reaction is rare [Schellekens, H., Immunogeniaty of therapeutic proteins: clinical implications and future prospects. Clin Ther, 2002. 24(11): p. 1720-40; discussion 1719]. Many factors influence the generation of an immune response, be it the presence of non-human amino acid sequences within the antibody structure, the nature of the target molecule, route of application or the type of disease where the therapeutic is indicated. Within two charges of the same product, which contain exactly identical amino acid primary sequences, the formation of aggregates, the presence of differentially-folded 3-dimensional structures of the protein, or the presence of impurities may severely affect immunogenicity [Schellekens, H., When biotech proteinsgo off-patent. Trends Biotechnol, 2004. 22(8): p. 406-10]. The dramatic consequences of immunogenicity are impressively observed when anti-product antibodies cross-react with an endogenous protein of the patient, which was observed after a formulation change of recombinant erythropoietin-alpha [Casadevall, N., Antibodies against rHuEPO: native and recombinant. Nephrol Dial Transplant, 2002. 17 Suppl 5: p. 42-7; Casadevall, N., Pure red cell aplasia and anti-erythropoietin antibodies in patients treated with epoetin. Nephrol Dial Transplant, 2003. 18 Suppl 8: p. viii37-41; Casadevall, N. and L. Croisille, [Erythropoiesis disorders and other central autoimmune anemias]. Rev Prat, 2001. 51(14): p. 1547-51; Casadevall, N., et al., Pure red-cell aplasia and antierythropoietin antibodies in patients treated with recombinant erythropoietin. N Engl J Med, 2002. 346(7): p. 469-75]. Thus, systems to monitor the consequences manufacturing changes may have on the quality of the product in terms of relative immunogenicity are urgently needed.

Changes in primary sequence of a protein can be readily monitored using *in vitro* systems consisting of isolated human dendritic cells and human T-helper lymphocytes [Stickler, M.M., D.A Estell, and F.A Harding, CD4+ T-cell epitope determination using unexposed human donor peripheral blood mononuclear cells. J Immunother, 2000. 23(6): p. 654-60]. These models may be limited by donor-to-donor variance in T-cell precursor frequency and the polymorphic nature of the MHC, necessitating the investigation of large numbers of donors with different MHC genotype to arrive at statistically meaningful results. Most importantly, more subtle changes excluding primary but involving a protein's tertiary structure cannot be monitored in isolated *in vitro* systems and necessitate more complete models of the immune system. Such models can be provided by *in vivo* animal systems.

Inter alias, human homologs are immunogenic in animals, and as such, testing human antibodies in animals is hardly predictive of their immunogenic potential in humans [Schellekens, H., Bioequivalence and the immunogenicity of biopharmaceuticals. Nat Rev Drug Discov, 2002. 1(6): p. 457-62]. However, the relative immunogenicity of different preparations may be assessed in some instances [Palleroni, A.V., et al., Interferon immunogenicity: preclinical evaluation of interferon-alpha 2a. J Interferon Cytokine Res, 1997. 17 Suppl 1: p. S23-7]. While human proteins are foreign to animals and will thus evoke an immune response, animals that have been made transgenic for the human protein of interest will recognize the transgene as self and will become tolerant towards the protein encoded by the transgene. While no animal model or in-vitro system can presently predict immune responses against a therapeutic protein in humans, which can exclusively and reliably only be determined in clinical trials, human antibody-transgenic mice [Storb, U., Transgenic mice with immunoglobulin genes. Annu Rev Immunol, 1987. 5: p. 151-74] are the best model to evaluate the factors responsible for breaking B-cell tolerance against therapeutic antibodies, and will thus constitute an important tool in the assessment of immunocomparability, i.e. the effect manufacturing changes may have on the relative immunogenic properties of a therapeutic antibody in transgenic mice.

Antibody-transgenic mice are well-known in the art; however, they have not been employed to solve the problem of immunocomparability assessment of therapeutic antibodies. The present invention addresses the shortcomings encountered within human *in vitro* systems and offers a solution to the presently unsolved problem of ensuring immunological comparability of therapeutic antibodies.

The present invention provides a method for assessing the immunocomparability of antibodies and thereby allows to determine whether an antibody preparation elicits an immune response or not relative to a standard antibody preparation (relative immunogenicity).

The present invention provides a method for determining the relative immunogenicity of an exogenous antibody comprising the following steps:
a) creating a non-human animal, which is transgenic for a standard antibody,
b) contacting said transgenic non-human animal with an exogenous antibody, whereby said exogenous antibody is a variant of the transgenic standard antibody, and
c) determining the immune response of the transgenic non-human animal elicited by the exogenous antibody of (b).

The exogenous antibody may be a therapeutic antibody. Preferably, the exogenous antibody is a human, humanized or chimeric antibody. More preferably, the transgenic antibody is an immunoglobulin gamma (IgG). Even more preferably, the antibody is the antibody against the human amyloid beta peptide or a variant thereof. The most preferred antibody is a variant of anti-Abeta IgG. The anti-Abeta IgG is described in detail in the patent application WO 03/070760, which is herewith fully incorporated as reference.

Preferably, the transgenic standard antibody is a human, humanized or chimeric antibody. More preferably, the transgenic antibody is an immunoglobulin gamma (IgG). Even more preferably, the antibody is the antibody against the human amyloid beta peptide. The most preferred antibody is anti-Abeta IgG.

The transgenic non-human animal may be any non-human animal. The preferred non-human animal is a mammal. More preferably, the non-human animal is a rodent such as a mouse or a rat.

The term "standard antibody" as used herein refers to an antibody generated under defined conditions, which serves as an acknowledged measure of comparison for quantitative or qualitative value.

The term "variant" or "antibody variant" as used herein refers to an antibody that differs in structural characteristics, the preparation method, formulation, or storage conditions from a standard antibody. The structural variants may comprise variation of the primary, secondary and tertiary protein structure (i.e. conformational changes), glycosylation and chemical modifications of amino acids. Further structural variations are i.e. alternative inter-domain constructs (VL/VL, VH/VH), dimers, oligomers and larger aggregates.

Factors with possible influence on relative immunogenic properties may comprise structural alterations of drug substance, formulation, impurities and contaminants of drug product. Of particular interest are differences in the immunogenicity between native protein and structural variants which might arise from changes in downstream processing, storage, formulation and handling [Sharma, V.K and D.S. Kalonia, Temperature- and pH-induced multiple partially unfolded states of recombinant human interferon-alpha2a: possible implications in protein stability. Pharm Res, 2003. 20(11): p. 1721-9; Chirino, A.J. and A Mire-Sluis, Characterizing biological products and assessing comparability following manufacturing changes. Nat Biotechnol, 2004. 22(11): p. 1383-91]. These variations comprise alternations in primary structure like exchange of amino acids, glycosylation variants and chemical modifications like acetylation, asparagine-deamidation, methionin, histidine or tryptophan oxidation or other chemical modifications caused by for instance oxidative or light stress, excipients or impurities with the potential to induce derivatizations [Moss, C.X., et al., Asparagine deamidation perturbs antigen presentation on class II MHC molecules. J Biol Chem, 2005; Mamula, M.J., et al., Isoaspartyl post-translational modification triggers autoimmune responses to self-proteins. J Biol Chem, 1999. 274(32): p. 22321-7; Duenas, E.T., et al., Comparison between light induced and chemically induced oxidation of rhVEGF. Pharm Res, 2001. 18(10): p. 1455-60; Gribben, J.G., et al., Development of antibodies to unprotected glycosylation sites on recombinant human GM-CSF. Lancet, 1990. 335(8687): p. 434-7; Mimura, Y., et al., Role of oligosaccharide residues of IgG1-Fc in Fc gamma RIIb binding. J Biol Chem, 2001. 276(49): p. 45539-47; Chianese-Bullock, K.A., et al., Antigen processzng of two H2-IEd-restricted epitopes is differentially influenced by the structural changes in a viral glycoprotein. J Immunol, 1998. 161(4): p. 1599-607; Khossravi M, Shire SJ, Borchardt RT. Evidence for the involvement of histidineA(12) in the aggregation and precipitation of human relaxin induced by metal-catalyzed oxidation. Biochemistry. 2000 May 16;39(19):5876-85; Margiloff L, Chaplia L, Chow A, Singhal PC, Mattana J. Metal-catalyzed oxidation of immunoglobulin G impairs Fc receptor-mediated binding to macrophages. Free Radic Biol Med. 1998 Nov 1;25(7):780-5; Duenas, E.T., et al., Comparison between light induced and chemically induced oxidation of rhVEGF. Pharm Res, 2001. 18(10): p. 1455-60]. Secondary and tertiary structure variants can arise for instance as consequence of modifications in the primary structure, by full or partial reduction, variation of intramolecular disulfide bridging ("scrambling"), or intramolecular covalent crosslinking. Conformers with primary sequence identical with the native protein can be formed for instance by thermal stress, a brief shift of pH or incubation with organic solvents [Sharma, V.K and D.S. Kalonia, Temperature- and pH-induced multiple partially unfolded states of recombinant human interferon-alpha2a: possible implications in protein stability. Pharm Res, 2003. 20(11): p. 1721-9; Vermeer, A.W. and W. Norde, The thermal stability of immunoglobulin: unfolding and aggregation of a multi-domain protein. Biophys J, 2000. 78(1): p. 394-404; Ronnelid, J., et al., Immune complexes from SLE sera induce IL10 production from normal peripheral blood mononuclear cells by an FcgammaRII dependent mechanism: implications for a possible viaous cycle maintaining B cell hyperactivity in SLE. Ann Rheum Dis, 2003. 62(1): p. 37-42; Melnikova, Y.I., et al., Antigen-binding activity of monoclonal antibodies after incubation with organic solvents. Biochemistry (Mosc), 2000. 65(11): p. 1256-65; Reed MA, et al., The denatured state under native conditions: a non-native-like collapsed state of N-PGK. J Mol Biol. 2006 Mar 24;357(2):365-72]. Strucural variations of higher order like alternative inter-domain constructs (VL/VL, VH/VH), dimers, oligomers and larger aggregates can be formed by covalent intermolecular crosslinking, induced for instance by oxidative stress, and/or the presence of excipients or impurities with the potential to induce intermolecular crosslinking. Dimers, oligomers and larger aggregates can also be formed by non-covalent intermolecular interactions for instance as consequence of formation of non-native conformers, micelle formation or modifications in the primary structure.

Of special interest are larger aggregates because of their repetitive epitope characteristics, in addition to conformational changes, which are known to increase the immunogenicity of various therapeutic proteins (Braun, A., et al., Protein aggregates seem to play a key role among the parameters influencing the antigeniaty of interferon alpha (IFN-alpha) in normal and transgenic mice. Pharm Res, 1997. 14(10): p. 1472-8; Hochuli, E., Interferon immunogenicity: technical evaluation of inteferon-alpha 2a. J Interferon Cytokine Res, 1997. 17 Suppl 1: p. S15-21; Hermeling S, et al., Structural characterization and immunogenicity in wild-type and immune tolerant mice of degraded recombinant human interferon alpha2b. Pharm Res. 2005 Dec;22(12):1997-2006). Aggregate formation is known to be promoted by for instance exposure to light, physical stress (shaking or stirring) and can occur during long-term storage especially under elevated temperatures or by inappropriate handling of drug products (physical stress) (Wang, W. Protein aggregation and its inhibition in biopharmaceutics. Int J Pharm. 2005 Jan 31, 289(1-2):1-30, and citations herein).

The term "immune response" as used herein refers to any response of the immune system to an antigenic stimulus, including antibody production, cell-mediated immunity, and immunological tolerance.

The term "therapeutic antibody" as used herein refers to a monoclonal antibody which may be used in treatment, therapy and diagnosis of disease. The therapeutic antibody may an antibody produced by any animal as immune response to an antigen. Preferably, the therapeutic antibody is produced by a rodent, more preferably, by a mouse. The therapeutic antibody may also an antibody produced by genetic engineering. Suitable methods are well known in the art, i.e. phage display (Brekke OH and Loset GA, New technologies in therapeutic antibody development Curr Opin Pharmaco. 2003 Oct: 3(5): 544-50.). Preferably, the therapeutic antibody is a human antibody, a humanized antibody or a chimeric antibody.

The present invention further provides a method for assessing the immunocomparability of a therapeutic antibody, comprising the following steps:
a) creating a non-human animal which is transgenic for a standard antibody,
b) immunizing said transgenic non-human animal with the therapeutic antibody wherein the therapeutic antibody is a variant of the standard antibody,
c) collecting the sera of the immunized transgenic animal,
d) measuring the antibodies in the sera directed against the therapeutic antibody.

The antibodies in the sera directed against the therapeutic antibody are antibodies produced by the transgenic animal as an immune response against the therapeutic antibody.

Methods for producing a transgenic non-human animal are well known in the art. Suitable methods are i.e. in Hogan B., Beddington R., Costantini F. & Lacy E. Manipulating the mouse embryo. A laboratory manual. 2nd Edition (1994). Cold Spring Harbor Laboratory Press.

The expression of the antibody in the transgenic non-human animal may be constitutive or inducible. Preferably, the expression of the antibody is constitutive.

Methods for assessing the immune response are well known in the art. A suitable method is for example ELISA; further methods may be found in Harlow E. & Lane D. Antibodies. A laboratory manual. (1988) Cold Spring Harbor Laboratory Press.

Furthermore, the present invention relates to a non-human animal transgenic for a standard antibody and its use for determining the relative immunogenicity of an exogenous antibody wherein the exogenous antibody is a variant of the transgenic standard antibody, preferably of a therapeutic antibody.

The transgenic non-human animal may be any non-human animal. The preferred non-human animal is a mammal. More preferably, the non-human animal is a rodent such as a mouse or a rat. Methods for producing a transgenic non-human animal are well known in the art. Suitable methods are i.e. in Hogan B., Beddington R., Costantini F. & Lacy E. Manipulating the mouse embryo. A laboratory manual. 2nd Edition (1994). Cold Spring Harbor Laboratory Press.

Methods for assessing the immune response are also well known in the art. A suitable method is for example ELISA; further methods may be found in Harlow E. & Lane D. Antibodies. A laboratory manual. (1988) Cold Spring Harbor Laboratory Press.

The exogenous antibody may be a therapeutic antibody. Preferably, the exogenic antibody is a human, humanized or chimeric antibody. More preferably, the transgenic antibody is an immunoglobulin gamma (IgG). Even more preferably, the antibody is the antibody against the human amyloid beta peptide or a structural variant thereof. The most preferred antibody is a variant of anti-Abeta IgG. The anti-Abeta IgG is described in detail in the patent application WO 03/070760 which is herewith fully incorporated as reference.

Preferably, the transgenic standard antibody is a human, humanized or chimeric antibody. More preferably, the transgenic antibody is an immunoglobulin gamma (IgG). Even more preferably, the antibody is the antibody against the human amyloid beta peptide. The most preferred antibody is anti-Abeta IgG.

Having now generally described this invention, the same will become better understood by reference to the specific examples, which are included herein for purpose of illustration only and are not intended to be limiting unless otherwise specified, in connection with the following figures.

### Figures:

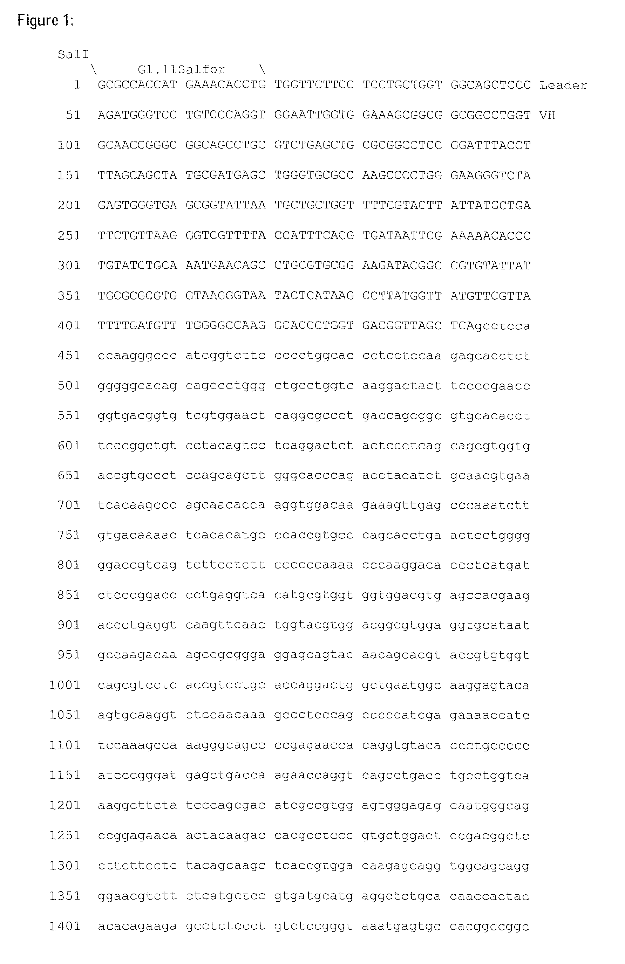

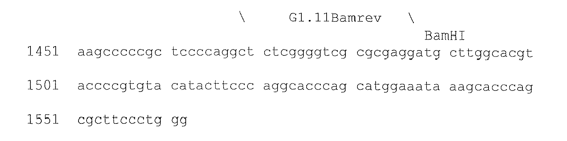

Figure 1 shows the entire coding sequence of the human Ig γ1 gene specific for human Aβ as cloned into the expression vector pHSE3' for transgenic expression. The position and the name of the primers used in PCR amplification are displayed above or below the corresponding sequence. The Stop codon is shown in bold.

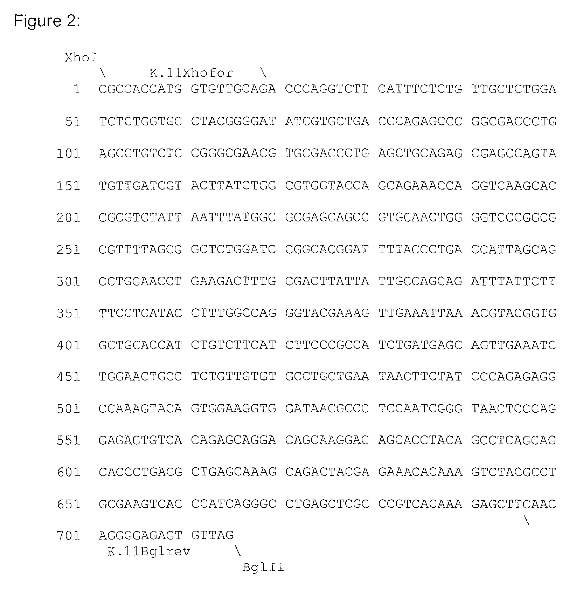

Figure 2 shows the entire coding sequence of the human Ig κ gene specific for human Aβ as cloned into the expression vector pHSE3' for transgenic expression. The position and the name of the primers used in PCR amplification are displayed above or below the corresponding sequence. The Stop codon is shown in bold.

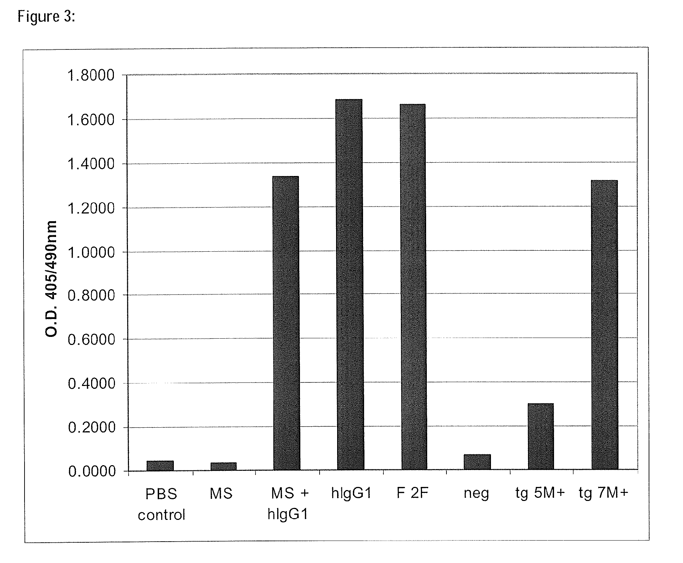

Figure 3 shows a graphical representation of the serum analysis of transgenic mice. Sandwich ELISA specific for human kappa light chain and human gamma heavy chain was performed. MS: mouse serum. hIgG1: recombinant human immunoglobulin of gamma 1 isotype. F 2F: founder mouse 2F. Neg: PCR-negative littermate control. Tg 5M+: transgenic mouse 5M+. Tg 7M+: transgenic mouse 7M+. Transgenic mice express both antibody chains within the same molecule.

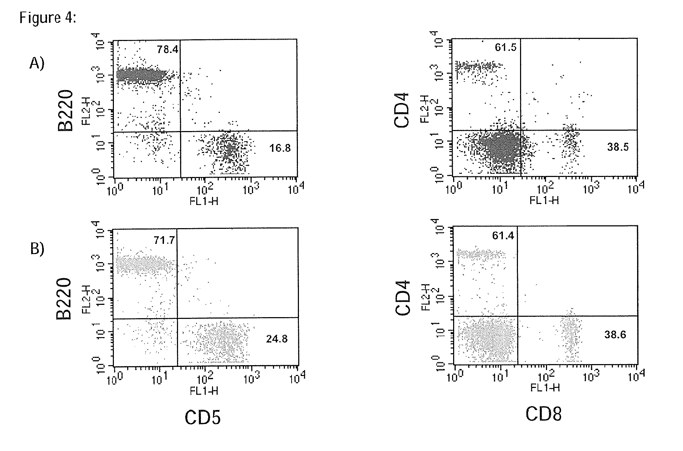

Figure 4 shows a graphical representation of the assessment of lymphocyte subpopulations in FACS (Fluorescence activated cell sorting). Peripheral blood lymphocytes from wildtype and transgenic mice were stained with antibodies specific for B-cells, T-cells and T-helper as well as cytotoxic T-cells. No significant differences between lymphocyte sub-populations of wild-type and hIgG1-transgenic mice can be detected. A) wildtype Bl/6, B) transgenic mice.

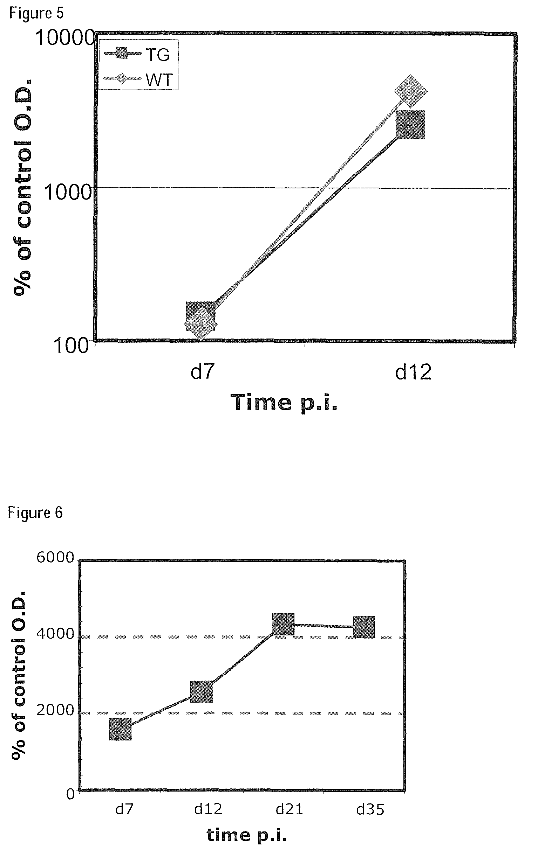

Figure 5 shows a graphical representation of the anti-KLH antibody titer in wildtype (WT) and transgenic (TG) mice. Wild-type and transgenic mice were immunized with KLH on day 0. On day 7 and day 12, anti-KLH antibody titers were assessed. Transgenic mice respond similar to wild-type mice upon KLH challenge.

Figure 6 shows a graphical representation of anti-HUMIRA titers in transgenic mice. Transgenic mice were immunized with the human IgG1 antibody HUMIRA on day 0. On days 7, 12, 21 and 35, serum an ti-HUMIRA titers were measured. Transgenic mice generate a response against HUMIRA.

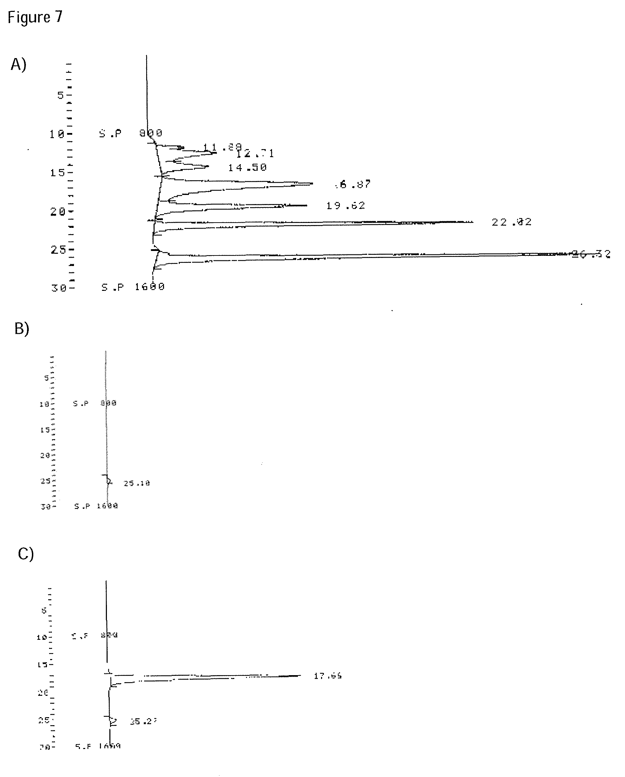

Figure 7 shows Size Exclusion Chromatograms (SEC) of A) molecular weight standard B) Mab11 placebo and C) Mab11. Equipment, working conditions and procedure were as described in the method section.

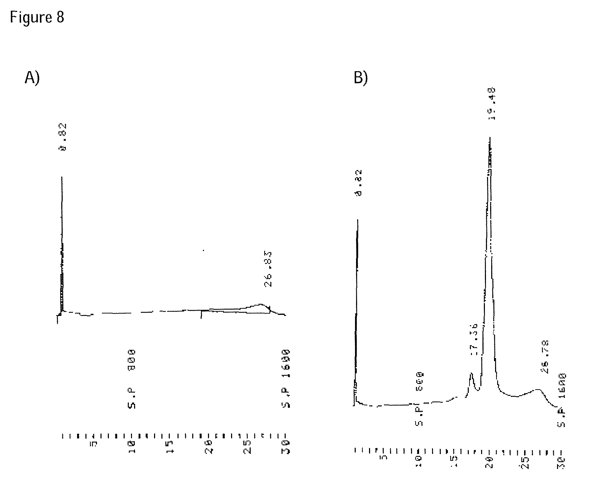

Figure 8 shows Ion Exchange Chromatograms of A) Mab11 placebo and B) Mab11.
Equipment, working conditions and procedure were as described in the method section.

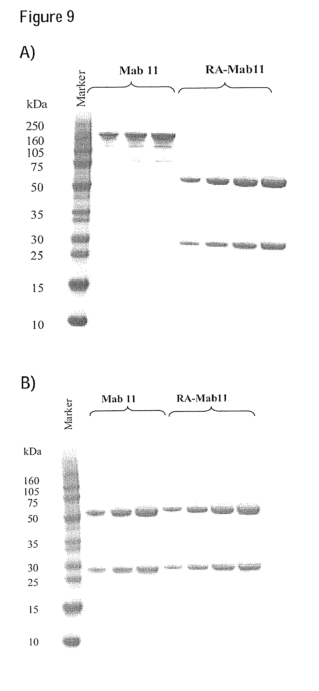

Figure 9 shows a picture SDS-PAGE of Mab11 and reduced/alkylated Mab11 (RA-Mab11) on 4-12 % Bis-Tris Gels run under A) non-reducing B) reducing conditions.

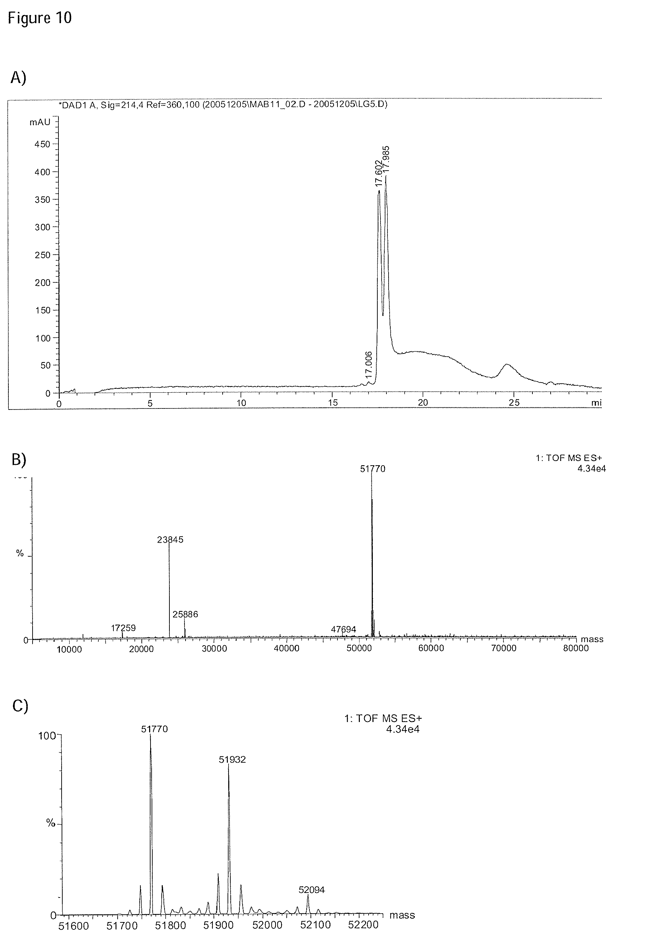

Figure 10 shows a graphical representation of LC/MS analysis of reduced/alkylated Mab11 (RA-Mab11). A) C8 RP-HPLC, UV-trace (214nm) B) deconvoluted mass-spectra. C) Zoomed region of the HMW mass peak in B). For assignment of observed and calculated masses see Table 3.

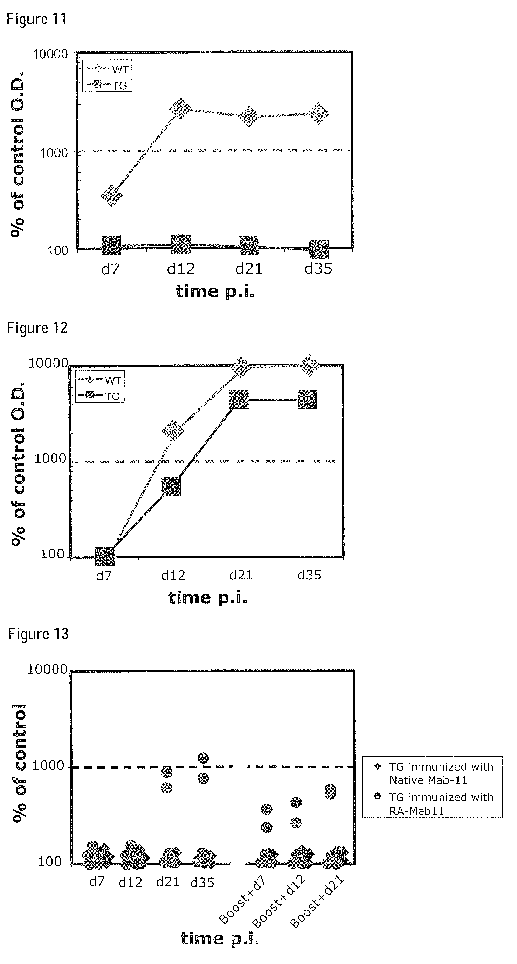

Figure 11 shows a graphical representation of anti-Mab-11 titers in wildtype (WT) and transgenic (TG) animals. Wild-type and transgenic mice were immunized with native Mab-11 on day 0. On day 7, 12, 21 and 35, anti-Mab-11 antibody titers were assessed. Transgenic mice are tolerant to Mab-11 challenge.

Figure 12 shows a graphical representation of anti-RA Mab-11 titers (RA= reduced-alkylated) in wildtype (WT) and transgenic (TG) mice. Wild-type and transgenic mice were immunized with RA Mab-11 on day 0. On days 7, 12, 21 and 35, anti-RAMab-11 titers were assessed. Both wild-type and transgenic mice respond vs. RA Mab-11.

Figure 13 shows a graphical representation of antibody responses of transgenic (TG) mice to Mab-11 ( 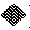 ) and Reduced-Alkylated (RA)-Mab-11 ( 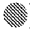 ). Transgenic mice were immunized with native Mab-11 and RAMab-11. 3 months after the first immunization, animals were boosted with native Mab-11. Antibody responses vs. native Mab-11 were assessed before and after boosting. 2/5 mice show cross-reaction with native Mab-11.

### Examples:

Commercially available reagents referred to in the examples were used according to manufacturer's instructions unless otherwise indicated.

### Example 1: Generation of mice transgenic for human immunoglobulin

### Generation of construct

A cDNA encoding an immunoglobulin (Ig) heavy chain (H) of the isotype γ1 (SEQ. ID. NO: 1) and a cDNA encoding a light chain (L) of the isotype κ (SEQ. ID. NO: 2) and with specificity for human Ab peptide were used [Bardroff, M.e.a., *Anti-amyloid beta antibodies and their use.* EP03001759 EP, 2003]. The cDNAs were amplified in a PCR reaction using the primers in Table 1. The 5'primers contain a SalI (or compatible XhoI) site and the 5'primers contain a BamHI (or compatible BglII) site for directed insertion into the pHSE3' vector [Pircher, H., et al., T cell tolerance to Mlsa encoded antigens in T cell receptor V beta 8.1 chain transgenicmice. Embo J, 1989. 8(3): p. 719-27.]. The PCR-amplified cDNAs were first enzymatically cut with both restriction enzymes SalI and BamHI, and then individually inserted into the corresponding sites of the vector pHSE3'. The expression of the Ig cDNAs in pHSE3' is driven by the murine promoter of the MHC class I gene H-2k and enhanced by the murine Ig H gene enhancer located 3' of the cloned genes [Pircher, H., et al., T cell tolerance to Mlsa encoded antigens in T cell receptor V beta 8.1 chain transgenic mice. Embo J, 1989. 8(3): p. 719-27.]. This expression vector ensures high levels of production of the corresponding inserted gene products in T and B lymphocytes of the transgenic mice ([Pircher, H., et al., T cell tolerance to Mlsa encoded antigens in T cell receptor V beta 8.1 chain transgenic mice. Embo J, 1989. 8(3): p. 719-27.] and unpublished observations). The entire expression cassette, including (from 5' to 3'): the H-2k promoter, the inserted cDNA, the poly-A and splice sites and the Ig H gene enhancer element, was then excised from the vector by means of restriction enzyme cut with XhoI and agarose gel purification, and prepared in an adequate concentration for microinjection into fertilized mouse oocytes (2 µg/ml in 10 mM TrisHCl/0.1 mM EDTA, pH 7). The coding potential of the cDNA was confirmed by sequencing the entire cDNAs encoding the anti-Aβ Ig H and L genes (see figure 1).

**Table 1. Sequences of cloning primers.**

| Primer name | Sequence | Restriction site (in bold) | SEQ. ID |
|---|---|---|---|
| G1.11Salfor (5'IgH) | 5'-ACGTGTCGACGCCGCCACCATGAAACACCTG-3' | SalI (GTCGAC) | 3 |
| G1.11Bamrev (3'IgH) | 5'-ACGTGGATCCTCATTTACCCGGAGACAG-3' | BamHI (GGATCC) | 4 |
| K.11Xhofor (5 IgL) | 5'-ACGTCTCGAGGCCGCCACCATGGTGTTGCAG-3' | XhoI (CTCGAG) | 5 |
| K.11Bglrev (3'IgL) | 5'-ACGTAGATCTCTAACACTCTCCCCTGTTG-3' | BglII (AGATCT) | 6 |

### Generation of anti-Aβ IgG1 transgenic mice

Fertilized oocytes obtained from C57BL/6 female donors were microinjected with a 1:1 mixture of the purified XhoI fragments encoding for the IgH and L genes described in the previous section to obtain double transgenic animals. Pups born from these microinjected embryos were screened for the presence of the transgenes by amplifying genomic DNAprepared from tail biopsies with specific primers. The primers used are indicated in Table 2.

**Table 2. Primer sequences for detection of transgenes.**

| PCR assay | Primers | | PCR fragment | SEQ. ID. |
|---|---|---|---|---|
| Ig H gene | 5H2KP | 5'-ATGAATTCACAGTTTCACTTCTGCACC-3' | 660 bp | 7 |
| | G1.0501 | 5'-TGTACTCCTTGCCATTCAGC-3' | | 8 |
| Ig L gene | 5H2KP | 5'-ATGAATTCACAGTTTCACTTCTGCACC-3 | 660bp | 9 |
| | K.44 | 5'-GCTCATCAGATGGCGGGAAG-3' | | 10 |

The PCR reaction was performed using 1µl (about 100 ng) of total DNA obtained from the tail biopsy, in PCR reaction with the following conditions: 1 min at 90°, 30x [10 sec at 94°, 30 sec at 64°, 90 sec at 72°], 7 min at 72°. The PCR-amplified DNA fragments of about 660 bp were finally visualized in 1.5 % agarose gels separately for the transgenic IgH and L genes.

In total, 5 double transgenic mice bearing both the transgenic IgH and L genes and 1 single transgenic mouse bearing only the IgH gene were generated and bred.

### Example 2: Phenotypic characterization of transgenic mice

### Serum analysis

Blood was obtained by tail vain puncture. Coagulation was performed overnight at room temperature. Serum was separated by centrifugation at 500xg for 10' and frozen at - 20°C until further analysis.

To determine whether transgenic mice express fully human antibodies, an ELISA system was developed. Human antibodies were captured using a polyclonal goat-anti-human kappa-chain specific antibody (Sigma K 3502). Detection was performed with a monoclonal mouse-anti-human gamma-chain specific antibody coupled to peroxidase (POD, Sigma A 0170). As shown in figure 5, transgenic mice express fully human immunoglobulin.

Lymphocyte subpopulations were measured in FACS. Blood was obtained by tail vein punture and collected in heparin-coated tubes (Sarstedt). Erythrocytes were lysed in lysis buffer (Buffer EL, Cat. No. 70217, Qiagen) as described by the manufacturer. Cells were resuspended in FACS-buffer (PBS, 0.05% NaN₃, 3% FCS), antibody staining was performed according to the manufacturer's suggestion (Becton-Dickinson). As shown in fig. 4, both wild-type and transgenic mice contain comparable ratios of B-cells (B220+), T-cells (CD5+), CD4+ T-helper cells and CD8+ cytotoxic T-cells. Therefore, expression of transgenic human IgG1 does not affect lymphocyte subpopulations.

For assessment of immunological competence, groups of 5 transgenic and wild-type littermate control mice were immunized with the model antigen KLH (Keyhole Limpet Hemocynin, Sigma, H-7017, Lot 121K4838). Anti-KLH titers were measured in ELISA, using KLH coated to maxisorp plates (Nunc) for capture and anti-mouse IgG1 (BD Pharmigen) for detection. Wild-type and transgenic mice mount a comparable immune response to KLH (fig. 5). Thus, hIgG1-transgenic mice are immuno-competent.

The response against a closely related antigen was assessed by immunization with the human IgG1 antibody HUMIRA (Abbott). 10µg of HUMIRA were emulsified in 200µl of Rehydragel HPA (Reheis). Animals were immunized intraperitonial (i.p.) on day 0, blood was drawn by tail vain punture on days 7, 12, 21 and 35, serum was prepared, and anti-HUMIRA titers were assessed by ELISA, using HUMIRA for capture by coating to maxisorp plates (Nunc) and detecting anti-HUMIRA antibodies by anti-mouse IgG (BD Pharmigen). As shown in Fig. 6, transgenic mice mount a response against the closely related human IgG1 antibody HUMIRA.

### Example 3: Production purification and characterization of Mab11

### Cell culture

Chinese hamster ovary cells (Hoffmann-La Roche, Switzerland) were adapted to serum-free growth in the in house DHI media formulation (Invitrogen), which is a mixture of DMEM, Ham's F12 and IMDM in the respective proportions of 1:1:2 (v:v:v) (Schlaeger & Schumpp 1992) with the plant hydrolysates of *0.2% soy HyPep 1510 and 0.2% rice HyPep* 4601 (Kerry Bioscience). The cells were routinely grown in spinner flasks (Bellco, Inotech AG, Dotlikon, Switzerland) with 80-100 rpm using about 70% of the recommended working volume. The large scale culture and transfection was either performed in 6L spinner flasks or 5 to 14 liter stirred tank bioreactors (Chemap, MBR, Zürich, and Infors, Bottmingen, Switzerland). Plasmid preparations for pMAb-11 were performed using a commercially available kit (Nucleobond Ax, Macherey-Nagel AG, Switzerland). The plasmid concentrations were determined spectrophotometrically and estimated by agarose gel electrophoresis with pUC18 DNA as standard (Pharmacia Biotech, Zürich, Switzerland).

### Transfection procedure

For transfection experiments, suspension CHO cells grown in DHI media were seeded in at a density of 1x10e6/ml in the final vessel and cultured to a density of 2-3x10e6 cells/ml (1-2d). Then the cell concentration was again adjusted to 1-1.5x10e6 cells/ml with fresh DHI media and the culture was grown between 3-4h before the transfection took place. After addition of the transfection complexes, the cells were cultured at 37°C for 24h post transfection. From 24h onwards, the temperature was shifted to 33°C for the remainder of the culture of 5-6 days. Supernatant samples were routinely harvested every 24h and monoclonal antibody (mAb) content was determined by Protein A affinity chromatography (Amersham, Aekta FPLC system). The supernatant was harvested via filtration and loaded directly onto the first purification columns with Protein A resin (mAb Select, Amersham, GE).

### Preparation of transfection complexes

The DNA complexes were formed in 1/10 of the culture volume in HL medium without heparin at room temperature (Schlaeger & Schumpp 1990, Schlaeger 1996). Under optimized gene delivery conditions for CHO cells, 0.7 µg DNA per ml cells was added to 0.1 ml fresh medium and mixed gently. After two minutes, 1 µl Ro1539 Xtreme Gene (Roche Applied Science, Indianapolis, USA) was added and mixed. After incubating 15 minutes at room temperature, the transfection complex was transferred to the equivalent amount of cells, i.e. in the bioreactor or spinner flask.

### Isolation and purification of Mab11 from fermentation supernatant

All procedures were performed under endotoxin free conditions by using tempered glassware only, sanitization of all equipment including columns was done with 0.5M NaOH and sterilfiltration of all buffers (0.22µm). Only fresh gelmaterial was used.

### First step: Protein A affinity chromatography

Mab Select gel (Amersham) in a BioRad econo column (BioRad) was first washed with 3 gel volumes (GV) equilibration buffer (25 mM Tris/HCl; 25 mM NaCl, 5 mM EDTApH 7.1), 2GV regeneration buffer (2M Guanidin, 100 mM Tris pH 7.5) followed by 5 GV equilibration buffer. After the pre-run, max. 20 mg Mab/ml were loaded onto the gel. The column was first washed with 3 GV equilibration buffer, then with 4 GV washing buffer (1 M Tris/HCl pH 7.2) and 3 GV equilibration buffer. Protein A bound antibody was eluted with 100mM HAc pH 2.9. For virus inactivation, the eluate is adjusted to pH ≤ 3.6 with HAc and then incubated for 15 min. at RT then adjusted with 1M Tris to pH 4.0.

### Second step: Ion Exchange Chromatography (Cation exchange chromatography)

SP-Toyopearl 650M (Tosoh) in a BioRad econo column (BioRad) was first washed with 2 GV regeneration buffer (0.5 M NaOH, 1M NaCl) and 3GV regeneration buffer buffer A (50 mM HAc, pH 5.0). After the pre-run, max. 10 mg Mab/ml were loaded onto the gel. The column was first washed with 0.5 GV buffer A, then a gradient elution from 0 to 100% buffer B (50mM HAc, 1M NaCl pH 5.0) was performed. Eluting protein fractions were collected and analyzed by IEX and SEC HPLC.

### Third step: Size Exclusion Chromatography (Flow-though anion exchange chrom atography)

Fractions of the second step were concentrated by ultrafiltration and adjusted to pH 7.5 and loaded on Q-Sepharose FF gel (Biorad) in a BioRad econo column (BioRad). SEC chromatography was performed using 25mM Tris/HCl, 80 mM Na-acetate, pH 7.5 as mobile phase. The effluent was fractionated according to the UV signal and adjusted to pH 5.5. Exchange of buffer to Mab11 placebo containing 20 mM Histidine/140 mM NaCl, pH 5.5 and adjustment of concentration was done in an Amicon stirred cell (Amicon) using a 10 kDa membrane. The solution was finally filtered over a 0.22 µm Millex-GV sterile filter (Millipore) and stored in aliquots at -80°C.

### Characterization of Mab11

### Size Exclusion Chromatography

Samples of purified Mab11 were analyzed by size exclusion chromatography by using the Jasco PU-980 HPLC system. The samples were chromatographed on a TSK-Gel G3000SWXL, 7.8 x 300 mm, 5µm column (Tosho Biosciences) with 0.2M K₂HPO₄, 0.25M KCl, pH 7.0 as mobile phase. The flow rate was set at 0.5 ml/min. The absorbance was monitored at 220 nm using a Jasco UV-975 detector connected to a Merck-Hitachi D-2500 recording system.

The column was equilibrated until a stable baseline was obtained. A sequence was injected corresponding gel filtration standard (BioRad, 151-1901, including 670 kD bovine thyroglobulin, 158 kD bov. IgG, 44 kD OVA, 17 kD eq. myoglobin, 1.35 kD vit.B₁₂), Mab11 placebo (negative control: buffer without Mab11), Mab11 sample. Injected amount corresponded to approximately 50 µg of sample. Representative Size Exclusion Chromatograms are shown in Fig. 7. Results: Symmetrical peak at retention time corresponding 155-160 kDa. No aggregates or fragments were detectable.

### Ion Exchange Chromatography

Samples of purified Mab 11 were analyzed by ion exchange chromatography using the Jasco PU-980 HPLC system. The samples were chromatographed on a Mono S 5/50 GL column (Amersham Biosciences) by using a gradient from 0 % B to 52 % B in 20 min. (Mobile Phase A: 50mM malonic acid/malonate in water, pH5.3; Mobile Phase B: 1 M Na-acetate in Mobile Phase A, pH5.3). The flow rate was set at 1 ml/min. The absorbance was monitored at 280 nm using a Jasco UV-975 detector connected to a Merck-Hitachi D-2500 recording system.

The column was equilibrated with Mobile Phase A until a stable baseline was obtained. A sequence was injected corresponding Mab11 placebo and Mab11 sample. Injected amount of Mab 11 corresponded to approximately 50 µg. Representative Ion Exchange Chromatograms (IEC) are shown in Fig. 8. Result: 95% of Mab11 sample elutes as single peak with non-resolved shoulder at higher retention time. About 5% elute with shorter retention time.

### Sodium Dodecyl Sulfate Polyacrylamide Gel Electrophoresis

Samples of purified Mab11 were analyzed by SDS-PAGE using the Xcell Mini-Cell II Gelsystem (Invitrogen). Pre-diluted samples were mixed with reducing or non-reducing sample buffer to concentrations of 2-8 µg per 20 µL Reducing sample buffer was prepared by mixing NuPAGE LDS sample buffer (Invitrogen) with NuPAGE reducing agent (Invitrogen) according manufacturers' advice. Samples in reducing sample buffer were incubated for 10 minutes at 70°C. Samples and standard were loaded on a NuPAGE4-12 % Bis-Tris Gel, 10 well-comb, 1.0 mm thickness (Invitrogen, #NP0301BOX). As standard, Mark 12^{™} Molecular Weight Marker (Invitrogen, #LC5677) covering a range of 200-2.5kDa was used. The gels were run at 200 V for 1h with NuPAGE MOPS SDS running buffer (Invitrogen). Gels were stained over night with StainEase Gel staining tray (Invitrogen), scanned and analyzed by densitometry. Protein concentrations were calculated referring to a standard sample curve obtained from the reference standard run in the same gel. Results: SDS-PAGE under reducing conditions shows two bands corresponding to molecular weights of IgG₁ H- and L-chains. No aggregates or fragments detectable (see Fig. 9).

### Mass spectrometry

For mass spectrometrical analysis, reduced/carboxymethylated antibody was prepared according to the methods described in example 5. Samples were subjected to an analytical RP-HPLC analysis by using the Agilent 1100 Capillary-HPLC system. The samples were chromatographed on an Agilent Poroshell C8-reversed-phase (0.5 x 75 mm) column by using a gradient system (A: water 0.1 % formic acid, B: acetonitrile 0.1 % formic acid). The flow rate was set at15 µl/min and the column temperature was set to 70°C. The chromatography stream was subsequently subjected to the ESI ion source of the mass spectrometer. Measurements were performed on an ESI-Q-TOF mass spectrometer (Micromass/Waters Q-TOF Ultima, Manchester, UK) in the positive mode with lockspray mass correction. Protein spectra were deconvolutet with the Masslynx MaxEnt1 module.

LC/MS analysis of RA-Mab11 is shown in Fig. 10, observed and calculated masses are assigned in Table 3.

**Table 3. Results LC/MS analysis of RA-Mab11, assignments of observed masses (see Fig. 10). L-Chain = light chain, H-Chain= heavy chain.**

| Observed mass [M+H]⁺ | Assignment (theoret. mass [M+H]⁺) |
|---|---|
| 23845 Da | Mab 11; L-Chain, reduced & carboxymethylated (23844 Da) |
| 51770 Da | Mab11; H-Chain-G₀, Pyro-Glu, - Lys, reduced & carboxymethylated (51770 Da) |
| 51932 Da | Mab11; H-Chain-G₁, Pyro-Glu, - Lys, reduced & carboxymethylated (51932 Da) |
| 52094 Da | Mab11; H-Chain-G₂, Pyro-Glu, - Lys, reduced & carboxymethylated (52094 Da) |

Results: The detected masses match with the theoretically expected for reduced/carboxymethlated H- and L-chain of an Antibody with Mab11 primary sequence with one pyro-Glu and with one Lys missing in the H-chain. The H-chain is mainly G₀-and G₁ glycosylated, G₂ only to a minor extend.

### Example 4: Immunological assessment of tolerance.

Groups of 5 transgenic and wild-type littermate control mice were immunized with 10µg recombinant Mab-11 emulsified in 200µl of Rehydragel-HPA (Reheis) via the i.p. route. Blood was obtained by tail vain puncture on days 7, 12, 21 and 35. Serum was prepared by coagulation, serum anti-Mab-11 titers were measured in ELISA by coating with Mab-11 on maxisorp plates (Nunc), and detecting anti-Mab-11 antibodies by anti-mouse IgG (BD Pharmingen). ELISA analysis revealed the generation of a robust immune response against Mab-11 in the wild-type animals, while hIgG1-transgenic animals were unable to mount an immune response to rhIgG1 (fig. 11). Thus, hIgG1 transgenic mice are tolerant towards exogenously supplied antibody of the same isotype and specificity, e.g. towards the product of their transgene.

### Example 5: Generation of variants of drug substance.

As structural variants, reduced/alkylated Mab11 was chosen, because the derivatives are well-defined and appropriate for comprehensive physicochemical characterization. By reductive cleavage of the interchain disulfide bonds the antibody is cleaved into heavy and light chains. Free sulfhydryl groups were subsequently alkylated with iodoacetic acid to prevent polymerisation by reoxidation.

### Production and characterization of reduced/alkylated Mab11

For the preparation of fully reduced and carboxymethylated Mab11, dithiothreitol (DTT) was dissolved in deionized water to 0.1g/ml and added to 1mg/ml Mab11 in 6M Guanidine HCl 0.3M Tris/HCl pH 8.5 to a final concentration of 20 mM. After 60 min. incubation at 50°C ⁺/₋ 3°C, freshly dissolved iodoacetic acid (IAA) 0.33g/ml in deionized water was added to a final concentration of 50mM. The alkylation proceeded in the dark at room temperature for 30 min. IAA was inactivated by addition of a 1.5-fold molar excess of DTT relative to IAA and incubation for 10 min. at RT.

The reduced/alkylated antibody (RA-Mab-11) was purified using a PD-10 desalting column (Amersham) according to the manufacturer's advice with 20mM Tris/HCl buffer pH 8.5 as mobile phase, maximum sample load 1.5 ml per column. The protein-containing effluent was purified a second time using PD-10 or NAP-5 desalting columns (Amersham) with PBS pH 7.4 or Mab11-placebo as mobile phase. Protein-containing fractions were collected, sterile-filtered under a laminar flow, aliquoted in sterile polypropylene tubes and stored at -80°C. Product control and determination of protein concentration of reduced/carboxymethylated antibodies was performed by capillary LC-QTOF-MS and SDS-PAGE, as outlined in example 3.

Characterization of RA-Mab11, results: SDS-PAGE under reducing conditions shows two bands corresponding to molecular weights of IgG₁ H- and L-chains. No aggregates or fragments detectable (see Fig.9). Results of mass spectrometrical analysis see example 3 (Table 3 and Fig. 10).

### Example 6: Evaluation of immunogenicity of reduced/alkylated (RA-) variants of Mab-11 in Mab-11 transgenic mice and non-transgenic littermates

To determine whether conformational changes compared to the native protein make a critical contribution to sensitization in the in vivo transgenic mouse model, we immunized transgenic mice and non-transgenic littermates with derivatives of Mab-11 prepared by full or partial reduction and alkylation of disulfide bonds. The magnitude of humoral immune responses against native protein or structural variant was evaluated by ELISA.

Groups of wild-type and transgenic mice (N=5) were immunized on day 0 via the i.p. route, using 10 µg of RA-Mab-11 emulsified in 200µl of Rehydragel-HPA (Reheis). Blood was drawn by tail vein puncture on days 7, 12, 21 and 35. Serum was prepared by coagulation and stored at -20°C.

Antibodies binding to RA-Mab-11 were measured by ELISA. Wells of 96-well MaxiSorpTM plates (Nunc) were coated with RA- Mab-11 in PBS overnight at 4°C. Blocking was carried out with 2% BSA in PBS containing 0.05 % Tween 20 (PBST). PBST was used for washing and 0.5 % BSA in PBST was used as a diluent. After 3 washes, 100 µl of diluted serum samples were added to the coated wells and incubated for 1 h at 37°C. Anti-mouse IgG1 antibody (BD Pharmigen) was used to evaluate antibody levels. Binding of the antibody was detected following development with ABTS substrate solution (Roche) at 450 nm using a automated plate reader.

ELISA analysis revealed that RA-Mab-11 raised a strong humoral immune response in both Mab-11 transgenic mice and non-transgenic littermates (fig. 12), while Mab-11 transgenic mice were tolerant towards native, recombinant Mab-11 treatment (fig. 11). However, native Mab-11 induced an immune response in wild-type littermates (fig. 11). Interestingly, immunization with RA-Mab-11 broke tolerance against the native self protein in some animals (fig. 13), since mice developed cross-reacting antibodies against native Mab-11 upon immunization with RA-Mab-11. Boosting with native Mab-11 three months after the primary immunization induced a memory B-cell response characterized by crossreacting anti-RA-Mab-11, anti-Mab-11 titers in the same crossreacting mice detected before (fig. 13). These results clearly indicate that a structural variant of Mab-11 induced an immune response in transgenic mice, which are tolerant to the unaltered, native protein. Thus, Mab-11 transgenic mice are a suitable system to detect changes in the antibody's secondary and tertiary structure.

## Claims

1. A method for determining the relative immunogenicity of an exogenous antibody comprising
a) creating a non-human animal which is transgenic for a standard antibody,
b) contacting said transgenic non-human animal with an exogenous antibody whereby said exogenous antibody is a variant of the transgenic standard antibody,
c) determining the immune response of the transgenic non-human animal elicited by the exogenous antibody of (b).

2. The method according to claim 1, wherein the exogenous antibody is a human antibody, a humanized antibody or a chimeric antibody.

3. The method according to claim 1, wherein the transgenic standard antibody is a human antibody, a humanized antibody or a chimeric antibody

4. The method according to claim 3 wherein the transgenic standard antibody is an antibody against the human amyloid beta peptide.

5. The method according to any one of the claims 1 to 4, wherein the non-human animal is a mammal.

6. The method according to any one of the claims 1 to 4, wherein the non-human animal is a rodent.

7. The method according to any one of the claims 1 to 4, wherein the non-human animal is a mouse.

8. The method according to any one of the claims 1 to 6 wherein the variant comprise variation of the primary, secondary and tertiary protein structure, glycosylation and chemical modifications of amino acids.

9. A method for assessing the immunocomparability of a therapeutic antibody,
comprising
a) creating a non-human animal which is transgenic for a standard antibody,
b) immunizing said transgenic non-human animal with the therapeutic antibody wherein the therapeutic antibody is a variant of the standard antibody,
c) collecting the sera of the immunized transgenic animal,
d) measuring the antibodies in the sera directed against the therapeutic antibody.

10. Use of a non-human animal transgenic for a standard antibody for determining the relative immunogenicity of an exogenous antibody wherein the exogenous antibody is a variant of said transgenic standard antibody.

11. The use according to claim 10, wherein the exogenous antibody is a human antibody, a humanized antibody or a chimeric antibody.

12. The use according to claim 10 wherein the transgenic standard antibody is an antibody against the human amyloid beta peptide.

13. The use according to any one of the claims 10 to 12, wherein the non-human animal is a mammal.

14. The use according to any one of the claims 10 to 12, wherein the non-human animal is a rodent.

15. The use according any one of the claims 10 to 12, wherein the non-human animal is a mouse.

16. The methods and uses substantially as described herein before especially with reference to the foregoing examples.


### Annex to the application documents - subsequently filed sequences listing

SEQUENCE LISTING

<110> F. Hoffmann-La Roche

<120> In vivo model of immunocomparability

<130> 23082

<160> 10

<170> PatentIn version 3.3

<210> 1
<211> 1562
<212> DNA
<213> Homo sapiens

<400> 1
gcgccaccat gaaacacctg tggttcttcc tcctgctggt ggcagctccc agatgggtcc 60

tgtcccaggt ggaattggtg gaaagcggcg gcggcctggt gcaaccgggc ggcagcctgc 120

gtctgagctg cgcggcctcc ggatttacct ttagcagcta tgcgatgagc tgggtgcgcc 180

aagcccctgg gaagggtcta gagtgggtga gcggtattaa tgctgctggt tttcgtactt 240

attatgctga ttctgttaag ggtcgtttta ccatttcacg tgataattcg aaaaacaccc 300

tgtatctgca aatgaacagc ctgcgtgcgg aagatacggc cgtgtattat tgcgcgcgtg 360

gtaagggtaa tactcataag ccttatggtt atgttcgtta ttttgatgtt tggggccaag 420

gcaccctggt gacggttagc tcagcctcca ccaagggccc atcggtcttc cccctggcac 480

cctcctccaa gagcacctct gggggcacag cagccctggg ctgcctggtc aaggactact 540

tccccgaacc ggtgacggtg tcgtggaact caggcgccct gaccagcggc gtgcacacct 600

tcccggctgt cctacagtcc tcaggactct actccctcag cagcgtggtg accgtgccct 660

ccagcagctt gggcacccag acctacatct gcaacgtgaa tcacaagccc agcaacacca 720

aggtggacaa gaaagttgag cccaaatctt gtgacaaaac tcacacatgc ccaccgtgcc 780

cagcacctga actcctgggg ggaccgtcag tcttcctctt ccccccaaaa cccaaggaca 840

ccctcatgat ctcccggacc cctgaggtca catgcgtggt ggtggacgtg agccacgaag 900

accctgaggt caagttcaac tggtacgtgg acggcgtgga ggtgcataat gccaagacaa 960

agccgcggga ggagcagtac aacagcacgt accgtgtggt cagcgtcctc accgtcctgc 1020

accaggactg gctgaatggc aaggagtaca agtgcaaggt ctccaacaaa gccctcccag 1080

cccccatcga gaaaaccatc tccaaagcca aagggcagcc ccgagaacca caggtgtaca 1140

ccctgccccc atcccgggat gagctgacca agaaccaggt cagcctgacc tgcctggtca 1200

aaggcttcta tcccagcgac atcgccgtgg agtgggagag caatgggcag ccggagaaca 1260

actacaagac cacgcctccc gtgctggact ccgacggctc cttcttcctc tacagcaagc 1320

tcaccgtgga caagagcagg tggcagcagg ggaacgtctt ctcatgctcc gtgatgcatg 1380

aggctctgca caaccactac acacagaaga gcctctccct gtctccgggt aaatgagtgc 1440

cacggccggc aagcccccgc tccccaggct ctcggggtcg cgcgaggatg cttggcacgt 1500

accccgtgta catacttccc aggcacccag catggaaata aagcacccag cgcttccctg 1560

gg                                                                  1562

<210>  2
<211>  715
<212>  DNA
<213>  Homo sapiens

<400>  2
cgccaccatg gtgttgcaga cccaggtctt catttctctg ttgctctgga tctctggtgc      60

ctacggggat atcgtgctga cccagagccc ggcgaccctg agcctgtctc cgggcgaacg     120

tgcgaccctg agctgcagag cgagccagta tgttgatcgt acttatctgg cgtggtacca     180

gcagaaacca ggtcaagcac cgcgtctatt aatttatggc gcgagcagcc gtgcaactgg     240

ggtcccggcg cgttttagcg gctctggatc cggcacggat tttaccctga ccattagcag     300

cctggaacct gaagactttg cgacttatta ttgccagcag atttattctt ttcctcatac     360

ctttggccag ggtacgaaag ttgaaattaa acgtacggtg gctgcaccat ctgtcttcat     420

cttcccgcca tctgatgagc agttgaaatc tggaactgcc tctgttgtgt gcctgctgaa     480

taacttctat cccagagagg ccaaagtaca gtggaaggtg gataacgccc tccaatcggg     540

taactcccag gagagtgtca cagagcagga cagcaaggac agcacctaca gcctcagcag     600

caccctgacg ctgagcaaag cagactacga gaaacacaaa gtctacgcct gcgaagtcac     660

ccatcagggc ctgagctcgc ccgtcacaaa gagcttcaac aggggagagt gttag          715

<210>  3
<211>  31
<212>  DNA
<213>  Artificial

<220>
<223>  Description of artificial sequence: G1.11Salfor, primer for 5'
       end of the heavy chain of human anti-Abeta IgG1

<400>  3
acgtgtcgac gccgccacca tgaaacacct g                                    31

<210>  4
<211>  28
<212>  DNA
<213>  Artificial

<220>
<223>  description of artificial sequence: G1.11Bamrev, primer for 3'
       end of heavy chain of human anti-Abeta IgG1

<400>  4
acgtggatcc tcatttaccc ggagacag                                        28

<210>  5
<211>  31
<212>  DNA
<213>  Artificial

<220>
<223>  Description of artificial sequence: K.11Xhofor, primer for 5'
       end of the light chain of human anti-Abeta IgG1

<400>  5

acgtctcgag gccgccacca tggtgttgca g                                    31

<210>  6
<211>  29
<212>  DNA
<213>  Artificial

<220>
<223>  Description of artificial sequence: K.11Bglrev, primer for 3'
       end of light chain of the human anti-Abeta IgG1

<400>  6
acgtagatct ctaacactct cccctgttg                                      29

<210>  7
<211>  27
<212>  DNA
<213>  Artificial

<220>
<223>  Description of artificial sequence: 5H2KP, PCR primer for heavy
       chain gene of human anti-Abeta IgG1

<400>  7
atgaattcac agtttcactt ctgcacc                                        27

<210>  8
<211>  20
<212>  DNA
<213>  Artificial

<220>
<223>  Description of artificial sequence: G1.0501, PCR primer for
       heavy chain gene of human anti-Abeta IgG1

<400>  8
tgtactcctt gccattcagc                                                20

<210>  9
<211>  27
<212>  DNA
<213>  Artificial

<220>
<223>  Description of artificial sequence: 5H2KP, PCR primer for light
       chain gene of human anti-Abeta IgG1

<400>  9
atgaattcac agtttcactt ctgcacc                                        27

<210>  10
<211>  20
<212>  DNA
<213>  Artificial

<220>
<223>  Description of artificial sequence: K.44, PCR primer for light
       chain gene of human anti-Abeta IgG1

<400>  10
gctcatcaga tggcgggaag                                                20

SEQUENCE LISTING

<110>  F. Hoffmann-La Roche

<120>  In vivo model of immunocomparability

<130>  23082

<140>  EP 06115620.4
<141>  2006-06-19

<150>  EP 05105946.7
<151>  2005-06-30

<160>  10

<170>  PatentIn version 3.3

<210>  1
<211>  1562
<212>  DNA
<213>  Homo sapiens

<400>  1
gcgccaccat gaaacacctg tggttcttcc tcctgctggt ggcagctccc agatgggtcc      60

tgtcccaggt ggaattggtg gaaagcggcg gcggcctggt gcaaccgggc ggcagcctgc     120

gtctgagctg cgcggcctcc ggatttacct ttagcagcta tgcgatgagc tgggtgcgcc     180

aagcccctgg gaagggtcta gagtgggtga gcggtattaa tgctgctggt tttcgtactt     240

attatgctga ttctgttaag ggtcgtttta ccatttcacg tgataattcg aaaaacaccc     300

tgtatctgca aatgaacagc ctgcgtgcgg aagatacggc cgtgtattat tgcgcgcgtg     360

gtaagggtaa tactcataag ccttatggtt atgttcgtta ttttgatgtt tggggccaag     420

gcaccctggt gacggttagc tcagcctcca ccaagggccc atcggtcttc cccctggcac     480

cctcctccaa gagcacctct gggggcacag cagccctggg ctgcctggtc aaggactact     540

tccccgaacc ggtgacggtg tcgtggaact caggcgccct gaccagcggc gtgcacacct     600

tcccggctgt cctacagtcc tcaggactct actccctcag cagcgtggtg accgtgccct     660

ccagcagctt gggcacccag acctacatct gcaacgtgaa tcacaagccc agcaacacca     720

aggtggacaa gaaagttgag cccaaatctt gtgacaaaac tcacacatgc ccaccgtgcc     780

cagcacctga actcctgggg ggaccgtcag tcttcctctt ccccccaaaa cccaaggaca     840

ccctcatgat ctcccggacc cctgaggtca catgcgtggt ggtggacgtg agccacgaag     900

accctgaggt caagttcaac tggtacgtgg acggcgtgga ggtgcataat gccaagacaa     960

agccgcggga ggagcagtac aacagcacgt accgtgtggt cagcgtcctc accgtcctgc    1020

accaggactg gctgaatggc aaggagtaca agtgcaaggt ctccaacaaa gccctcccag    1080

cccccatcga gaaaaccatc tccaaagcca aagggcagcc ccgagaacca caggtgtaca    1140

ccctgccccc atcccgggat gagctgacca agaaccaggt cagcctgacc tgcctggtca    1200

aaggcttcta tcccagcgac atcgccgtgg agtgggagag caatgggcag ccggagaaca    1260

actacaagac cacgcctccc gtgctggact ccgacggctc cttcttcctc tacagcaagc    1320

tcaccgtgga caagagcagg tggcagcagg ggaacgtctt ctcatgctcc gtgatgcatg    1380

aggctctgca caaccactac acacagaaga gcctctccct gtctccgggt aaatgagtgc 1440

cacggccggc aagcccccgc tccccaggct ctcggggtcg cgcgaggatg cttggcacgt 1500

accccgtgta catacttccc aggcacccag catggaaata aagcacccag cgcttccctg 1560

gg 1562

<210> 2
<211> 715
<212> DNA
<213> Homo sapiens

<400> 2
cgccaccatg gtgttgcaga cccaggtctt catttctctg ttgctctgga tctctggtgc 60

ctacggggat atcgtgctga cccagagccc ggcgaccctg agcctgtctc cgggcgaacg 120

tgcgaccctg agctgcagag cgagccagta tgttgatcgt acttatctgg cgtggtacca 180

gcagaaacca ggtcaagcac cgcgtctatt aatttatggc gcgagcagcc gtgcaactgg 240

ggtcccggcg cgttttagcg gctctggatc cggcacggat tttaccctga ccattagcag 300

cctggaacct gaagactttg cgacttatta ttgccagcag atttattctt ttcctcatac 360

ctttggccag ggtacgaaag ttgaaattaa acgtacggtg gctgcaccat ctgtcttcat 420

cttcccgcca tctgatgagc agttgaaatc tggaactgcc tctgttgtgt gcctgctgaa 480

taacttctat cccagagagg ccaaagtaca gtggaaggtg gataacgccc tccaatcggg 540

taactcccag gagagtgtca cagagcagga cagcaaggac agcacctaca gcctcagcag 600

caccctgacg ctgagcaaag cagactacga gaaacacaaa gtctacgcct gcgaagtcac 660

ccatcagggc ctgagctcgc ccgtcacaaa gagcttcaac aggggagagt gttag 715

<210> 3
<211> 31
<212> DNA
<213> Artificial

<220>
<223> Description of artificial sequence: G1.11Salfor, primer for 5'end
of the heavy chain of human anti-Abeta IgG1

<400> 3
acgtgtcgac gccgccacca tgaaacacct g 31

<210> 4
<211> 28
<212> DNA
<213> Artificial

<220>
<223> description of artificial sequence: G1.11Bamrev, primer for 3'
end of heavy chain of human anti-Abeta IgG1

<400> 4
acgtggatcc tcatttaccc ggagacag 28

<210> 5
<211> 31
<212> DNA
<213> Artificial

<220>
<223>  Description of artificial sequence: K.11Xhofor, primer for 5' end
      of the light chain of human anti-Abeta IgG1

<400>  5
acgtctcgag gccgccacca tggtgttgca g                                    31


<210>  6
<211>  29
<212>  DNA
<213>  Artificial

<220>
<223>  Description of artificial sequence: K.11Bglrev, primer for 3' end
      of light chain of the human anti-Abeta IgG1

<400>  6
acgtagatct ctaacactct cccctgttg                                       29


<210>  7
<211>  27
<212>  DNA
<213>  Artificial

<220>
<223>  Description of artificial sequence: 5H2KP, PCR primer for heavy
      chain gene of human anti-Abeta IgG1

<400>  7
atgaattcac agtttcactt ctgcacc                                         27


<210>  8
<211>  20
<212>  DNA
<213>  Artificial

<220>
<223>  Description of artificial sequence: G1.0501, PCR primer for heavy
      chain gene of human anti-Abeta IgG1

<400>  8
tgtactcctt gccattcagc                                                 20


<210>  9
<211>  27
<212>  DNA
<213>  Artificial

<220>
<223>  Description of artificial sequence: 5H2KP, PCR primer for light
      chain gene of human anti-Abeta IgG1

<400>  9
atgaattcac agtttcactt ctgcacc                                         27


<210>  10
<211>  20
<212>  DNA
<213>  Artificial

<220>
<223>  Description of artificial sequence: K.44, PCR primer for light
      chain gene of human anti-Abeta IgG1

<400>  10
gctcatcaga tggcgggaag                                                    20